# EUROPEAN PATENT SPECIFICATION

(11) **EP 2 877 561 B1**
(45) Date of publication and mention of the grant of the patent: **09.03.2016**
(21) Application number: 13762904.4
(22) Date of filing: 02.07.2013
(51) Int. Cl.: C11B 1/02, C11B 1/10, C12P 7/64

(54) **PROCESS OF PRODUCTION OF OIL FROM MICROALGAE**
VERFAHREN ZUR GEWINNUNG VON ÖL AUS MIKROALGEN
PROCÉDÉ DE PRODUCTION D'HUILE À PARTIR DE MICRO-ALGUES

(43) Date of publication of application: 03.06.2015
(73) Proprietor: Bio.Te.Ma. S.r.l., 09100 Cagliari (IT)
(72) Inventor: CANEPA, Pietro, I-16031 Bogliasco (Genova) (IT)
(74) Representative: Biggi, Cristina
(86) International application number: PCT/IT2013/000182
(87) International publication number: WO 2015/001578

(56) References cited:
- WO-A1-2011/159682
- US-A1- 2010 236 137
- US-A1- 2010 261 918
- US-B2- 7 905 930

## Description

### FIELD OF THE INVENTION

The present invention relates to a process for producing algal biomass intended for the production of lipids (oil) and/or high added value substances. The lipids obtained with the process of the invention can be used for energy production (for example by combustion) or human nutrition (for example as palm oil substitutes) or in the forage industry as supplements. The high added value substances can be employed, for example, as active ingredients in the pharmaceutical industry or as dietary supplements.

### BACKGROUND ART

Present-day energy production plants use raw materials that are by now being depleted (for example, fossil raw materials) and, moreover, their combustion determines the emission of polluting gases.

For these reasons, in recent years research has shifted toward the development of biofuels deriving from renewable, less polluting sources.

Initially, technologies were developed for the production of biofuels (for example, biodiesel) based on the transformation of food crops, such as oleaginous plants (rapeseed, soybean and sunflower). So-called first generation biofuels have however encountered limits due to the production costs and the use of farmland taken away from the cultivation of products intended for food. Furthermore, if one assesses the fuel's entire life cycle with LCA (Life Cycle Assessment) techniques, the production of first generation biofuels from vegetable food sources in some cases requires an energy input that is higher than the energy yield obtainable from using them as an energy source.

There was subsequently a shift toward technologies for the production of so-called second generation biofuels, which use the waste products and residues of the forestry and agri-food industries as the primary source. This has attenuated the conflict with the food sector, but these technologies are still characterized by a negative energy balance. Moreover, such technologies have yet to reach full industrial maturity.

Following the development of first and second generation biofuels, there is presently a shift toward third generation technologies, including the ones which use microalgae as raw materials. Microalgae are in fact characterized by a high capacity to produce lipids (oils), above all in given growth conditions and if particular species are selected. In addition to having a high lipid content, algae can also be sources of high added value products in a context of integrated production aimed at rendering the main technology for producing and extracting the oil for energy purposes sustainable. The high added value products that can be obtained from algae (parallel to the oil) are omega-3, carotenoids, vitamins and other antioxidant biochemical products which can be marketed (with high intrinsic value) in the dietary supplements industry, pharmaceutical industry, cosmetic industry, etc. Moreover, after extraction, the algal biomass can be used in the husbandry industry (for example as animal feed) or as a fertilizer.

Another large advantage of microalgae cultivation is that this technology does not enter into competition either with food production or with the availability of farmland. The oil obtained from microalgae can be used as such for the production of energy by combustion, or else it can be transformed into biodiesel, for example by a process of transesterification.

The term algae includes macroalgae (also called marine algae) and microalgae, which are photosynthetic microorganisms with a simple structure that are capable of growing rapidly in different environmental conditions. Microalgae are moreover characterized by a high lipid content which can vary, according to species, from 20% to 70% and gives a oil extraction yield equivalent to that of oleaginous plants. It follows that the amount of energy obtainable from microalgae cultivation is higher than that obtainable from oleaginous plants, their weight being equal. Furthermore, as already underscored above, from the de-oiled biomass obtained after separation of the oil it is possible to obtain cellulosic fractions for the production of other energy products (such as ethanol and biogas) or high added value substances such as vitamins, carotenoids, etc. for the pharmaceutical industry or dietary supplements industry.

There are known plants for the production of biodiesel from microalgae which comprise a unit for cultivating the microalgae, a unit for separating the lipids or other substances from the microalgae biomass, and a step of transforming the lipids into biodiesel, for example by transesterification processes.

Numerous plants for cultivating microalgae are also known. Typically, microalgae are cultivated in the presence of light (photoautotrophic growth method), in closed reactors or in outdoor ponds depending on the environmental situation and the species of microalgae used.

Cultivation in outdoor ponds requires very large spaces in order to be able to accommodate a quantity of microalgae such as to ensure a sufficient production of oil. The space required for cultivation ponds is undoubtedly a disadvantage in that cultivation is possible only in given areas of the planet where such large spaces are available in the required environmental conditions. Moreover, outdoor cultivation requires high volumes of water, is subject to atmospheric conditions and possible contamination by algal pathogens. Microalgae can be cultivated in closed reactors, illuminated with artificial light, which makes it possible to occupy smaller spaces and avoid the drawbacks due to exposure to atmospheric conditions and possible external contaminations. This variant of the photoautotrophic growth method is certainly advantageous in terms of biomass yield compared to cultivation in an open pond, but still has some intrinsic limits in terms of yield.

Such limits are represented by the possibility of transmitting light under conditions of microalgae concentrations exceeding 1-3 g/l, according to algal species. In other words, when the microalgae grow and reach the concentrations indicated, light cannot pass through and growth is thus arrested. This precludes obtaining economically sustainable production yields.

The drawbacks of the photoautotrophic growth method can be overcome by using a heterotrophic growth method, which consists in cultivating microalgae under lightless conditions (hence under conditions of stress), and feeding them simple sugars that represent the energy source, as a replacement for light, which allows the microalgae to proliferate. The heterotrophic method enables the limits in the concentration obtainable with the photoautotrophic method to be overcome, arriving even at concentrations comprised between 14 and 130 g/l of microalgae.

To increase biomass productivity even further, mixed photoautotrophic-heterotrophic microalgae cultivation plants are also known.

For example, US 7,905,930 describes a process for the production of biodiesel from microalgae which can comprise a sequential photoautotrophic and heterotrophic growth of the microalgae with the possible addition of a sugar feed in the heterotrophic stage. In this patent, both the photoautotrophic and heterotrophic stages of growth take place in open ponds, covered like greenhouses if necessary, while the use of bioreactors is described as too costly.

The disadvantages of pond cultivation are thus evident: spaces occupied too large to be cost-effective and possibility of contamination by external agents that are potentially lethal for the algae. Covering the ponds with a greenhouse plant can certainly not ensure a sterility of cultivation, which is essential for the industrialization of the process; that is, it cannot prevent algal diseases from blocking production and causing unsustainable economic damage.

Moreover, the two-stage photoautotrophic and heterotrophic process of US 7,905,930 can envisage feeding the microalgae, during the heterotrophic stage, with a sugar source of a starchy, cellulosic, lignocellulosic or polysaccharidic nature deriving from various plant materials, such as, for example, corn, sugarcane, switchgrass, grass residues and trees.

All these plant sources must however undergo depolymerisation or hydrolysis in order to be transformed into simpler sugars before they can be supplied to the microalgae as nutrients. This obviously entails an expenditure of energy which reduces the cost-effectiveness of the production of energy, without considering that plants for the depolymerisation or hydrolysis of complex sugars, such as cellulose or lignocellulose, to simple sugars are not at present conveniently available from an industrial standpoint.

Therefore, US 7,905,930 seems to describe a two-stage photoautotrophic-heterotrophic process that is unlikely to be applicable on an industrial scale in any cost-effective manner in energy production.

The present invention aims to overcome the drawbacks of the mixed the photoautotrophic-heterotrophic processes of growth of the prior art, as described, for example, in US 7,905,930, by providing an innovative process for the cultivation of microalgae and a plant for carrying it out.

### SUMMARY OF THE INVENTION

The present invention relates to a process for the production of lipids (oil) from microalgae comprising the cultivation of microalgae by means of sequential photoautotrophic-heterotrophic growth, wherein in the heterotrophic stage the microalgae are fed by administration of a sugar feed deriving from sugar production waste or deriving from food or confectionery industry waste. The sugar feed deriving from sugar production waste can be for example molasses or bagasse obtained from the processing of sugarcane or sugar beet. The sugar feed deriving from food or confectionery industry waste can be for example the wastewater deriving from the fruit candying process, which has a sugar content (glucose and/or sucrose) comprised between 20% and 60%. The process of the invention can be integrated with the process of producing sugar from sugar beet or sugarcane or the production process of the food or confectionery industry. The process of the invention, in fact, uses a residual product of sugar manufacturing, molasses or bagasse, and/or a feed deriving from food or confectionery industry waste, such as, for example, the wastewater from fruit candying. The invention also relates to a plant for the production of lipids (oil) from microalgae which comprises at least two production lines set in parallel, each comprising:
- a first step, common to said at least two production lines, a so-called laboratory or inoculation step, comprising at least 2 reactors in which the microalgae are initially cultivated;
- a second so-called "nursery" step comprising at least 3 reactors of increasing volume, in which the microalgae are grown also under conditions of artificial light (photoautotrophic growth);
- a third so-called fermentation step, comprising at least 5 reactors of increasing volume, in which the microalgae are grown under lightless conditions (heterotrophic growth) with the administration of molasses, wherein
   the reactors of the second and third steps are connected to one another to ensure the passage of the microalgae from one step to the next.

The plant of the invention can also comprise a step downstream of the fermentation step, in which the lipids and other added value substances are separated from the algal biomass.

The plant of the invention can be integrated with a sugar production plant so as to thereby reduce the costs of transporting the waste matter, for example the molasses, used as a nutritive source for cultivating the microalgae. Alternatively, the plant of the invention can be integrated with a fruit candying plant in the event that candying wastewater is used as the feed.

The lipids obtained from process of the invention can be used to produce energy by direct combustion of the same or transformed into other biofuels. The lipids obtained with the process of the invention can also be destined for human nutrition as replacements, for example, of palm oil. The invention also relates to a process for the production of added value substances from the microalgae cultivated with the method of the invention, for example vitamins, proteins, substances with pharmaceutical activity, and substances that are useful as dietary supplements. An example of an added value substance is lutein, which can be used as a dietary supplement.

### BRIEF DESCRIPTION OF THE FIGURES

Fig. 1 shows a schematic representation of a preferred embodiment of the entire process of the invention.
Fig. 2 shows a preferred embodiment of the plant of the invention in which the reactors used in the various steps of the method are shown.

### DETAILED DESCRIPTION OF THE INVENTION

As mentioned above, the invention relates to a process for the production of lipids (oils) from microalgae comprising at least the following steps:
a) cultivating the microalgae under conditions of natural and/or artificial light (photoautotrophic growth) and, in sequence, under lightless conditions with the addition of a sugar feed deriving from the processing of sugarcane or sugar beet, for example molasses or bagasse, and/or a sugar feed deriving from food or confectionery industry waste, such as, for example, the wastewater from fruit candying (heterotrophic growth);
b) separating the lipids (oil) from the microalgae.

The microalgae preferably used for the process of the invention are selected from among the following strains: Botryococcus B. (BCB), Chlorella vulgaris (CLV), Nannochloropsis sp. (NCS), Nannochloris sp. (NNS), Haematococcus pluvialis (HAC), Chlorella Protothecoide (CLP). The algal strain preferably used for the purposes of the invention is Chlorella Protothecoide.

The algal strains listed above are microalgae capable of producing, above all under conditions of stress (for example, in the absence of light), an amount of lipids comprised between 20% and 70% of their weight, preferably between 30% and 50% of their weight.

These strains are also characterized by a good growth rate.

In the photoautotrophic cultivation step the microalgae grow in an aqueous culture environment with a normal intake of nutrients, for example nitrates, favoured by the exposure to light.

When the microalgae reach an algal concentration (algal biomass) comprised between 1 and 5 g/L, preferably between 2 and 3 g/L, the biomass is subjected to the subsequent step of heterotrophic growth, since such concentrations represent the growth limit under conditions of light. Beyond such concentrations, light no longer reaches the algal biomass in a diffuse manner and the microalgae thus stop growing.

The period of growth of the microalgae until the maximum concentrations indicated above are reached, in the photoautotrophic step, is comprised between 2 and 7 days.

In the heterotrophic growth step (also called fermentation step), the algal biomass is deprived of light and is thus subjected to a condition of stress in which the microalgae increase the production of lipid substances precisely for the purpose of confronting the unfavourable environmental situation. In order to facilitate growth under these conditions which are favourable for increasing the production of lipids, the microalgae are fed a source of simple sugars deriving from sugar production waste. Among these, molasses is particularly useful because it comprises an amount of simple sugars comprised between 50% and 70% by weight.

Molasses is a sugar waste product obtained from the processing of sugarcane or sugar beet and is available in large quantities, and at a low cost, in countries where sugar is produced, for example Brazil, India, China, Mexico, Russia, Turkey, Ukraine, Egypt, Cuba, etc. Molasses has the advantage of not needing to undergo further processing before being administered to the algal biomass, since the sugars contained in it are simple sugars. In particular, it is not necessary to subject molasses to degradation or depolymerization redactions as in the case, for example, of the sources of carbohydrates used in the prior art (US 7,905,930). This represents an undoubted advantage in terms of industrializing the process and in terms of the cost-effectiveness of the production of lipids extracted from the algal biomass. Before supplying the molasses as a nutrient to the microalgae, it is preferable to dilute it with water to bring the concentration of sugars to between 20% and 50% by weight, preferably between 25% and 35% by weight.

As an alternative to waste deriving from the production of sugar, it is possible to use, as a sugar feed, waste deriving from the food or confectionery industry, in particular the water from fruit candying, which has a content of sugar (sucrose and/or glucose) comprised between 20% and 60% by weight.

The dilution operation is carried out in order to render the molasses and/or candying water sufficiently *fluid* so that it can be pumped into the reactor where heterotrophic growth occurs. Moreover, the dilution also serves to control the optimal concentrations of simple sugars in the fermentation step, which are, for example, comprised between 30 and 60 g/L, preferably between 40 and 50 g/L. During fermentation, it is possible to reach concentrations of algal biomass of up to 130 g/L, preferably comprised between 14 and 130 g/L. The production of algal biomass is preferably proportional to the amount of sugar supplied, typically with a ratio of 1:2.

Under the stress conditions of the fermentation step, the concentration of lipids in the microalgae increases to values comprised between 20 and 70% by weight, preferably between 30% and 50% by weight.

The heterotrophic growth step lasts for a total time (from the beginning to the end of the step) preferably comprised between 10 and 20 days.

In a preferred embodiment, the process of the invention comprises an inoculation or laboratory step upstream of the photoautotrophic growth step. In the inoculation step, microalgae growth is started by working on small volumes of the same in an aqueous environment, in the presence of light, and supplying the necessary nutritive substances, for example nitrates. The duration of this step depends on the type of algae and can be estimated as 30-60 days. The final concentration of the algal biomass in this step is 0.05-0.2 g/l.

Once the inoculation step has ended, the algal biomass is subjected to the photoautotrophic growth step as described above.

The step of separating the lipids from the algae can optionally also comprise a step of extracting high added value substances such as vitamins, proteins, carotenoids, active ingredients of pharmaceutical interest, active ingredients to be used as dietary supplements, etc. A preferred example of an active ingredient of interest for the dietary supplements industry which can be extracted from the algal biomass is lutein.

The step of separating the lipids from the algal biomass preferably comprises a step of eliminating the excess water and a step of separating the oil from the algal biomass, preferably by means of pressing, extraction or other methods known in the art.

The step of eliminating the excess water can comprise pressing the biomass (preferably with a belt press) and drying the biomass to further eliminate any residual water.

After the step of separating the algal biomass to obtain the oil it is possible to carry out a step of extracting the high added value substances as indicated above.

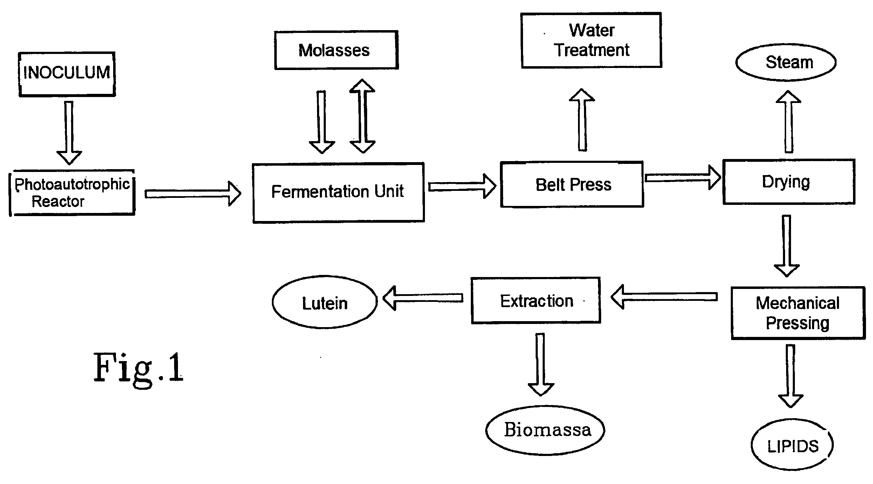

Figure 1 shows a block diagram of a preferred embodiment of the photoautotrophic-heterotrophic process of the invention.

The process of the invention is preferably a continuous process in which, after an initial growth of the microalgae in the inoculation step, when the biomass reaches a concentration comprised between 0.05 and 0.2 g/l, the algal biomass is transferred to the photoautotrophic growth step.

The algal biomass then undergoes growth in the presence of light and with a limited intake of nutrients, for example nitrates. When the biomass reaches concentrations comprised between 1 and 5 g/L, preferably between 2 and 3 g/L, it is transferred to heterotrophic step.

In the heterotrophic stage, growth of the biomass is intensified by light deprivation and the introduction of a sugar-based feed, molasses, deriving from the processing of sugar beet or sugarcane, or fruit candying water deriving from the confectionery industry. The molasses and water from fruit candying are diluted with water recovered from the belt press and/or drying step. Before the process water is recycled and reused to dilute the molasses, it undergoes a purification and sterilization treatment.

Once algal biomass concentrations comprised between 14 and 130 g/L are reached, the algal biomass is sent to the belt press for mechanical pressing and elimination of the water and the recycling thereof at the start of the process. The remaining water is further eliminated in the biomass drying step and the condensed steam further recycled at the start of the process. Once the excess water has been removed, the biomass is subjected to mechanical pressing to obtain the oil to be destined for the above-described uses. The de-oiled biomass can then be further used to extract high added value substances, such as, for example, lutein for the dietary supplements industry. The algal biomass, deprived of oil and the high added value substances, can be used, for example, for further energy production, for example by direct combustion.

The invention also relates to a plant for the production of lipids from microalgae which comprises at least two production lines set in parallel, each comprising:
- a first step, common to said at least due production lines, a so-called laboratory or inoculation step, comprising at least 2 reactors in which the microalgae are initially cultivated;
- a second so-called "nursery" step, in which the microalgae are grown under conditions of artificial light (photoautotrophic growth), comprising at least 3 reactors; and
- a third so-called fermentation step, in which the microalgae are grown under lightless conditions (heterotrophic growth) with the administration of molasses, comprising at least 5 reactors, wherein the reactors of the second and third steps are connected to one another to ensure the continuous passage of the microalgae from one step to the next.

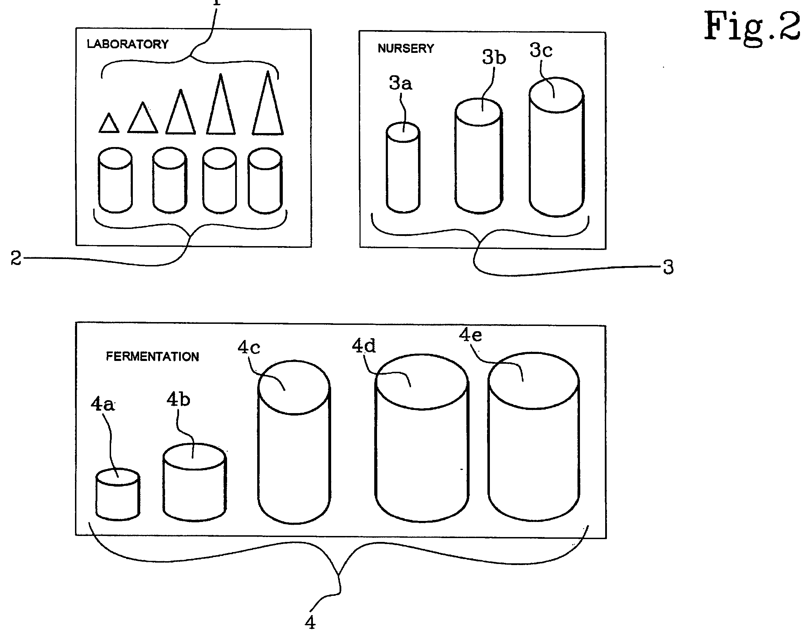

With reference to Figure 2, the laboratory step preferably comprises a series 1 of containers in which the growth of the algae is started off, followed by a series of reactors 2 with volumes increasing up to 30L. These reactors are not connected to the reactors of the nursery step; therefore, the transfer of the microalgae from the laboratory to the nursery reactors takes place by pouring; this is in order to avoid contamination and having to stop production. The transfer from the reactors of increasing volume of the laboratory step to the nursery reactors is carried out when the concentration of microalgae in said reactors reaches 0.02-0.5 g/L. The laboratory step is common to at least two production lines, i.e. it supplies at least two production lines in parallel, each comprising a nursery step and a fermentation step as described above. The production lines can advantageously be more than two, for example 10, hence the number can be increased according to the production needs of the plant. The laboratory step can thus supply 2 or more production lines. It is also possible to envisage 2 or more laboratory steps in the case in which the plant is particularly large, i.e. if the plant comprises a large number of production lines.

The second "nursery" step (where photoautotrophic growth takes place) comprises at least 3 reactors, (indicated together in Fig. 2 with the number 3) having a progressively larger volumetric capacity. Preferably, the reactors after the first one have a volumetric capacity which is double that of the preceding reactor.

Preferably, the first reactor, 3a, has a volumetric capacity comprised between 60 and 150 L. The second reactor, 3b, has a volumetric capacity ^preferably comprised between 150 and 300 L. The third reactor, 3c, has a volumetric capacity comprised between 300 and 600 L (as shown in Figure 2).

The at least three reactors are connected to one another, thus ensuring the transfer of the algal biomass from one reactor to the next larger one when the biomass concentration reaches a value comprised between 1 and 5 g/L, preferably between 2 and 3 g/L.

The reactors of this step are also equipped with a plant of artificial lighting to ensure the necessary amount of light for the photoautotrophic growth of the microalgae. The fermentation, or heterotrophic growth step comprises at least 5 reactors (indicated together in Fig. 2 with the number 4), including 3 reactors with increasing volumes and two reactors of equal volume.

The first 3 reactors with increasing volumes can comprise, for example, a first reactor 4a with a volume of 1,000 to 2,000 L (2 m³), a second reactor 4b with a volume of 10,000 to 20,000 L (20 m³) and a third reactor 4c with volume of 100,000 to 150,000 L (150 m³).

The third reactor simultaneously supplies the two final reactors with an equal volume. The quantity of microalgae is substantially divided into two and each of the two parts is sent to a final reactor (4d and 4e).

The 2 reactors with an equal volume, 4d and 4e, can have a volume comprised between 200,000 and 250,000 L (250 m³). These 2 reactors preferably work alternately, because one of the two is emptied at a time to collect the microalgae and send them on to the oil separation step.

The at least 5 reactors are connected to one another so as to permit the passage from one reactor to another when the concentration of algal biomass reaches the capacity limits of each reactor. The passage from one reactor to the next can take place, for example, after a period of time of between 2 and 8 days, so as to terminate the heterotrophic step in the production line 10-30 days after the start thereof.

These reactors are closed in such a way as to avoid the passage of light and are connected to a source of a sugar feed, for example molasses, bagasse or water from fruit candying.

The reactors of the second and third step are directly connected to one another to simplify and accelerate management of the process.

The plant envisages the installation of at least two production lines operating in parallel, wherein each of the steps described above comprises the same number of reactors as described above for one production line. The purpose of having at least two production lines set in parallel is to avoid having to stop production completely in the event that anomalies occur in microalgal growth, for example in the event of a disease of the microalgae, and to obtain a semi-continuous operation of the plant. The whole production line is preferably kept under conditions of sterility, hence in closed reactors, to avoid external contaminations that could compromise algal growth and/or the yield in terms of oil.

The plant of the invention can also comprise machinery for pressing the algal biomass (for example, a belt press), a dryer and machinery for mechanical pressing or extraction of the dried biomass to give the oil, placed downstream of the heterotrophic reactors.

The plant can also comprise a plant for recycling the water deriving from the pressing of the biomass and the condensed steam obtained from the drying of the pressed biomass.

Finally, the plant can also comprise a plant for extracting the high added value substances from the biomass, such as, for example, lutein.

The process and plant of the invention are preferably integrated with the process and plant for producing sugar from sugar beet or sugarcane.

The plant of the invention be placed, for example, in proximity to the sugar production plant so as to cut down the cost of transporting molasses to zero.

The distinctive features of the process and plant of the invention are those of:
- not necessarily using cultivatable areas, but rather industrial ones;
- using food industry waste as the raw material;
- obtaining a high yield of oil production both in percentage terms relative to the weight of the algae produced and in terms of Kg of oil per square metre of ground committed per year, typically 1,500 kg/m² per year;
- being realizable for production lines in parallel and thus implementable at different times depending on the availability of areas and raw materials;
- being autonomous from an energy standpoint with the combustion of the de-oiled biomass;
- not depending on environmental conditions.

## Claims

1. A process for the production of lipids (oils) from microalgae, comprising at least the following steps:
a) cultivating the microalgae under conditions of natural and/or artificial light (photoautotrophic growth) and, in sequence, under lightless conditions with the addition of a sugar feed obtained from waste from the processing of sugarcane or sugar beet selected from molasses or bagasse or a sugar feed deriving from food or confectionery industry waste (heterotrophic growth);
b) separating the lipids (oil) from the microalgae.

2. The process according to claim 1, wherein said sugar feed deriving from food or confectionery industry waste is the wastewater from fruit candying, comprising from 20% to 60% by weight of sugars.

3. The process according to any one of claims 1 to 2, wherein said microalgae are selected from among the following strains: Botryococcus B. (BCB), Chlorella vulgaris (CLV), Nannochloropsis sp. (NCS), Nannochloris sp. (NNS), Haematococcus pluvialis (HAC) and Chlorella Protothecoide (CLP).

4. The process according to any one of claims 1 to 3, wherein during photoautotrophic growth said microalgae reach a concentration comprised between 1 and 5 g/L, preferably between 2 and 3 g/L.

5. The process according to any one of claims 1 to 4, wherein said sugar feed, preferably molasses, is diluted with water, bringing the concentration of sugars to between 20% and 40% by weight, preferably between 25% and 35% by weight.

6. The process according to any one of claims 1 to 5, wherein during heterotrophic growth the concentration of algal biomass reaches 130 g/L; preferably, it is comprised between 14 and 130 g/L.

7. The process according to any one of claims 1 to 6, comprising an inoculation stage upstream of the photoautotrophic growth stage, wherein the growth of the microalgae is started.

8. The process according to claim 7, wherein the concentration of the algal biomass is comprised between 0.05 and 0.2 g/L.

9. The process according to any one of claims 1 to 8, wherein the step of separating the lipids from the microalgae comprises a stage of eliminating the excess water and a step of separating the oil from the algal biomass.

10. The process according to claim 9, wherein said step of eliminating the water comprises pressing the biomass and drying the biomass to further eliminate any residual water.

11. The process according to claim 9 or 10, wherein the step of separating the oil is followed by an extraction, from the residual biomass, of high added value substances, preferably vitamins, proteins, carotenoids, active ingredients of pharmaceutical interest and active ingredients to be used as dietary supplements.

12. A plant for the production of lipids (oil) from microalgae which comprises at least two production lines set in parallel, each comprising:
- a first step, common to said at least two production lines, a so-called laboratory or inoculation step, comprising at least 2 reactors in which the microalgae are initially cultivated;
- a second so-called "nursery" step, in which the microalgae are grown under conditions of artificial light (photoautotrophic growth), comprising at least 3 reactors; and
- a third so-called fermentation step, in which the microalgae are grown under lightless conditions (heterotrophic growth) with the administration of molasses, comprising at least 5 reactors,
wherein the reactors of the second and third steps are connected to one another to ensure the passage of the microalgae from one step to the next.

13. The plant according to claim 12, wherein said laboratory step comprises a series of containers in which the growth of the algae is started up, followed by a series of reactors with a volume increasing up to 30L.

14. The plant according to claim 12 or 13, wherein said nursery step comprises at least 3 reactors having a progressively greater volumetric capacity.

15. The plant according to any one of claims 12 to 14, wherein said at least 3 reactors of the nursery step have a volumetric capacity comprised, respectively, between 60 and 150 L, 150 and 300L and 300 and 600L.

16. The plant according to any one of claims 12 to 15, wherein said fermentation step comprises at least 5 reactors, 3 reactors with an increasing volume and 2 reactors of equal volume.

17. The plant according to claim 16, wherein said first 3 reactors comprise a first reactor with a volume of 1,000 to 2,000 L, a second reactor with a volume of 10,000 to 20,000 L and a third reactor with a volume of 100,000 to 150,000 L; said 2 reactors have a volume comprised between 200,000 and 250,000L.

18. The plant according to any one of claims 12 to 17, wherein said at least 5 reactors of the fermentation step are closed to prevent the passage of light and are connected to a source of a sugar feed, preferably molasses.

## Patentansprüche

1. Verfahren zur Gewinnung von Lipiden (Öl) aus Mikroalgen, umfassend mindestens folgende Schritte:
a) Kultivieren der Mikroalgen unter Bedingungen natürlichen und/oder künstlichen Lichts (photoautotrophes Wachstum) und anschließend unter lichtlosen Bedingungen mit dem Zusatz einer Zuckerzuführung, erhalten aus Abfall aus der Verarbeitung von Zuckerrohr oder Zuckerrüben, ausgewählt aus Melasse oder Bagasse, oder einer Zuckerzuführung aus der Nahrungsmittel- oder Süßwarenindustrie (heterotrophes Wachstum);
b) Trennen der Lipide (Öle) aus den Mikroalgen.

2. Verfahren nach Anspruch 1, wobei die Zuckerzuführung aus Nahrungsmittel- oder Süßwarenindustrieabfall das Abwasser aus dem Kandieren von Früchten ist, umfassend einen Gewichtsanteil von 20 bis 60 % Zucker.

3. Verfahren nach einem der Ansprüche 1 bis 2, wobei die Mikroalgen ausgewählt sind aus folgenden Stämmen: Botryococcus B. (BCB), Chlorella vulgaris (CLV), Nannochloropsis sp. (NCS), Nannochloris sp. (NNS), Haematococcus pluvialis (HAC) und Chlorella Protothecoide (CLP).

4. Verfahren nach einem der Ansprüche 1 bis 3, wobei die Mikroalgen während des photoautotrophen Wachstums eine Konzentration erreichen, eingeschlossen zwischen 1 und 5 g/l, vorzugsweise zwischen 2 und 3 g/l.

5. Verfahren nach einem der Ansprüche 1 bis 4, wobei die Zuckerzuführung, vorzugsweise Melasse, mit Wasser verdünnt wird, was die Zuckerkonzentration auf einen Wert zwischen 20 und 40 % Gewichtsanteil bringt, vorzugsweise zwischen 25 und 35 % Gewichtsanteil.

6. Verfahren nach einem der Ansprüche 1 bis 5, wobei die Konzentration der Algenbiomasse während des heterotrophischen Wachstums 130 g/l erreicht, vorzugsweise zwischen 14 und 130 g/l umfasst ist.

7. Verfahren nach einem der Ansprüche 1 bis 6, umfassend eine Inokulationsstufe vor der Stufe des photoautotrophen Wachstums, in der das Wachstum der Mikroalgen gestartet wird.

8. Verfahren nach Anspruch 7, wobei die Konzentration der Algenbiomasse zwischen 0,05 und 0,2 g/l umfasst ist.

9. Verfahren nach einem der Ansprüche 1 bis 8, wobei der Schritt zum Trennen der Lipide aus den Mikroalgen eine Stufe zum Beseitigen des überschüssigen Wassers und eine Stufe zum Trennen des Öls aus der Algenbiomasse umfasst.

10. Verfahren nach Anspruch 9, wobei der Schritt zum Beseitigen von Wasser das Pressen der Biomasse und das Trocknen der Biomasse umfasst, um zusätzlich jegliches Restwasser zu beseitigen.

11. Verfahren nach Anspruch 9 oder 10, wobei auf den Schritt zum Trennen der Öle eine Extraktion von Substanzen mit hohem Mehrwert aus der Restbiomasse folgte, vorzugsweise von Vitaminen, Proteinen, Carotinoiden, Wirkstoffen pharmazeutischen Interesses und Wirkstoffen, die als Ernährungsergänzungsstoffe verwendet werden können.

12. Anlage zur Herstellung von Lipiden (Ölen) aus Mikroalgen, die mindestens zwei parallel angeordnete Produktionslinien umfasst, von denen eine jede umfasst:
- einen ersten Schritt, der den mindestens zwei Produktionslinien gemeinsam ist und bei dem es sich um einen sog. Labor- oder Inokulationsschritt handelt, umfassend mindestens 2 Reaktoren, in denen die Mikroalgen anfänglich kultiviert werden;
- einen zweiten sog. "Zuchtschritt", in dem die Mikroalgen unter Bedingungen künstlichen Lichts (photoautotrophes Wachstum) gezüchtet werden, umfassend mindestens 3 Reaktoren, und
- einen dritten sog. Fermentationsschritt, in dem die Mikroalgen unter lichtlosen Bedingungen gezüchtet werden (heterotrophes Wachstum), mit der Administration von Melasse, umfassend mindestens 5 Reaktoren, wobei die Reaktoren des zweiten und dritten Schritts miteinander verbunden sind, um die Übergabe der Mikroalgen vom ersten Schritt zum nächsten zu garantieren.

13. Anlage nach Anspruch 12, wobei der Laborschritt eine Reihe von Behältern umfasst, in denen das Wachstum der Algen aufgenommen wird, gefolgt von einer Reihe von Reaktoren mit einem steigenden Volumen bis 30 l.

14. Anlage nach Anspruch 12 oder 13, wobei der Zuchtschritt mindestens 3 Reaktoren umfasst, aufweisend eine zunehmend größere Volumenkapazität.

15. Anlage nach einem der Ansprüche 12 bis 14, wobei die mindestens 3 Reaktoren des Zuchtschritts eine Volumenkapazität aufweisen, jeweils umfasst zwischen 60 und 150 l, 150 und 300 1 und 300 und 600 l.

16. Anlage nach einem der Ansprüche 12 bis 15, wobei der Fermentationsschritt mindestens 5 Reaktoren umfasst, 3 Reaktoren mit einem steigenden Volumen und 2 Reaktoren mit gleichem Volumen.

17. Anlage nach Anspruch 16, wobei die ersten Reaktoren einen ersten Reaktor mit einem Volumen von 1000 bis 2000 1 umfassen, einen zweiten Reaktor mit einem Volumen von 10.000 bis 20.000 1 und einen dritten Reaktor mit einem Volumen von 100.000 bis 150.000 l, wobei die 2 Reaktoren ein Volumen umfasst zwischen 200.000 und 250.000 1 aufweisen.

18. Anlage nach einem der Ansprüche 12 bis 17, wobei die mindestens 5 Reaktoren des Fermentationsschritts geschlossen sind, um den Durchgang von Licht zu verhindern, und an eine Quelle einer Zuckerzuführung, vorzugsweise Melasse, angeschlossen sind.

## Revendications

1. Procédé de production de lipides (huiles) à partir de micro-algues, comprenant au moins les étapes suivantes :
a) cultiver les micro-algues sous lumière naturelle et/ou artificielle (croissance photoautotrophe) et, en séquence, en l'absence de lumière avec l'ajout d'une charge de sucre obtenue à partir de déchets résultant de la transformation de la canne à sucre ou de la betterave à sucre sélectionnée à partir de la mélasse ou de la bagasse ou une charge de sucre dérivant de déchets issus de l'industrie alimentaire ou de la confiserie (croissance hétérotrophe) ;
b) séparer les lipides (huile) des micro-algues.

2. Procédé selon la revendication 1, dans lequel ladite charge de sucre dérivant des déchets de l'industrie alimentaire ou de la confiserie est l'eau résiduaire provenant du confisage de fruits, présentant une teneur en sucre comprise entre 20 et 60 % en poids.

3. Procédé selon l'une quelconque des revendications de 1 à 2, dans lequel lesdites micro-algues sont sélectionnées parmi les souches suivantes : Botryococcus B. (BCB), Chlorella vulgaris (CLV), Nannochloropsis sp. (NCS), Nannochloris sp. (NNS), Haematococcus pluvialis (HAC) et Chlorella Protothecoides (CLP).

4. Procédé selon l'une quelconque des revendications de 1 à 3, dans lequel lors de la croissance photoautotrophe lesdites micro-algues atteignent une concentration comprise entre 1 et 5 g/L, de préférence entre 2 et 3 g/L.

5. Procédé selon l'une quelconque des revendications de 1 à 4, dans lequel ladite charge de sucre, de préférence, de la mélasse, est diluée avec de l'eau, amenant la concentration en sucre entre 20 et 40 % en poids, de préférence, entre 25 et 35 % en poids.

6. Procédé selon l'une quelconque des revendications de 1 à 5, dans lequel lors de la croissance hétérotrophe, la concentration de la biomasse algale atteint 130 g/L ; de préférence entre 14 et 130 g/L.

7. Procédé selon l'une quelconque des revendications de 1 à 6, comprenant une phase d'inoculation en amont de la phase de croissance photoautotrophe, dans lequel la croissance des micro-algues commence.

8. Procédé selon la revendication 7, dans lequel la concentration de la biomasse algale est comprise entre 0,05 et 0,2 g/L.

9. Procédé selon l'une quelconque des revendications de 1 à 8, dans lequel l'étape consistant à séparer les lipides des micro-algues comprend une phase consistant à éliminer l'excès d'eau et une étape consistant à séparer l'huile de la biomasse algale.

10. Procédé selon la revendication 9, dans lequel l'étape consistant à éliminer l'eau comprend le pressage et le séchage de la biomasse pour éliminer davantage toute eau résiduelle.

11. Procédé selon les revendications 9 ou 10, dans lequel l'étape consistant à séparer l'huile est suivie par une extraction, de la biomasse résiduelle, de substances à haute valeur ajoutée, de préférence des vitamines, des protéines, des caroténoïdes, des principes actifs d'intérêt pharmaceutique et des principes actifs à utiliser comme suppléments diététiques.

12. Installation pour la production de lipides (huile) à partir de micro-algues comportant au moins deux lignes de production installées en parallèle, chacune comprenant :
- une première étape, commune auxdites au moins deux lignes de production, une étape dite de laboratoire ou d'inoculation, comprenant au moins 2 réacteurs dans lesquels les micro-algues sont initialement cultivées ;
- une seconde étape dite de de « nurserie », dans laquelle les micro-algues sont cultivées sous lumière artificielle (croissance photoautotrophe), comprenant au moins 3 réacteurs ; et
- une troisième étape dite de fermentation, dans laquelle les micro-algues sont cultivées en l'absence de lumière (croissance hétérotrophe) avec l'administration de mélasse, comprenant au moins 5 réacteurs, dans laquelle les réacteurs des deuxième et troisième étapes sont reliés réciproquement pour garantir le passage des micro-algues d'une étape à l'autre.

13. Installation selon la revendication 12, dans laquelle ladite étape de laboratoire comporte une série de récipients dans lesquels la croissance des algues a commencé, suivie par une série de réacteurs avec un volume croissant jusqu'à 30 L.

14. Installation selon les revendications 12 ou 13, dans laquelle ladite étape de « nurserie » comporte au moins 3 réacteurs ayant une capacité volumétrique progressivement plus importante.

15. Installation selon l'une quelconque des revendications de 12 à 14, dans laquelle lesdits au moins 3 réacteurs de l'étape de « nurserie » possèdent une capacité volumétrique comprise, respectivement, entre 60 et 150 L, 150 et 300 L et 300 et 600 L.

16. Installation selon l'une quelconque des revendications de 12 à 15, dans laquelle ladite étape de fermentation comporte au moins 5 réacteurs, 3 réacteurs à volume croissant et 2 réacteurs de volume équivalent.

17. Installation selon la revendication 16, dans laquelle lesdits 3 premiers réacteurs comprennent un premier réacteur d'un volume de 1 000 à 2 000 L, un deuxième réacteur d'un volume de 10 000 à 20 000 L et un troisième réacteur d'un volume de 100 000 à 150 000 L ; lesdits 2 réacteurs ont un volume compris entre 200 000 et 250 000 L.

18. Installation selon l'une quelconque des revendications de 12 à 17, dans laquelle lesdits au moins 5 réacteurs de l'étape de fermentation sont fermés pour empêcher le passage de la lumière et sont reliés à une source de charge de sucre, de préférence, de la mélasse.
